(19) Europäisches Patentamt
European Patent Office
Office européen des brevets

(11) Veröffentlichungsnummer: **0 337 152**
**A1**

(12) # EUROPÄISCHE PATENTANMELDUNG

(21) Anmeldenummer: **89104761.5**

(22) Anmeldetag: **17.03.89**

(51) Int. Cl.4: **C07D 307/32 , C07D 307/42 , C07H 13/10 , C07H 7/02 , A61K 31/34 , A61K 31/71 , C12N 1/20 , C12P 17/04 , C12P 19/02 , //(C12P17/04, C12R1:465),(C12P19/02, C12R1:465),(C12N1/20, C12R1:465)**

Der Anmelder hat eine Erklärung nach Regel 28 (4) EPÜ (Herausgabe einer Probe nur an einen Sachverständigen) eingereicht. Eingangsnummer(n) der Hinterlegung(en): DSM 4349 , DSM 4355 , DSM 4200 , DSM 4211.

(30) Priorität: **22.03.88 DE 3809562**

(43) Veröffentlichungstag der Anmeldung: **18.10.89 Patentblatt 89/42**

(84) Benannte Vertragsstaaten: **BE CH DE FR GB IT LI NL**

(71) Anmelder: **HOECHST AKTIENGESELLSCHAFT**
**Postfach 80 03 20**
**D-6230 Frankfurt am Main 80(DE)**

(72) Erfinder: **Grabley, Susanne, Dr.**
**Hölderlinstrasse 7**
**D-6240 Königstein/Taunus(DE)**
Erfinder: **Wink, Joachim, Dr.**
**Bieberer Strasse 133**
**D-6050 Offenbach(DE)**
Erfinder: **Kühlein, Klaus, Prof. Dr.**
**Fasanenstrasse 41**
**D-6233 Kelkheim (Taunus)(DE)**
Erfinder: **Seibert, Gerhard, Prof. Dr.**
**Gläserweg 21**
**D-6100 Darmstadt(DE)**
Erfinder: **Hütter, Klaus, Dr.**
**Robert-Scholz-Strasse 3**
**D-6232 Bad Soden am Taunus(DE)**
Erfinder: **Uhr, Hermann, Dr.**
**Düsseldorfer Strasse 67**
**D-5090 Leverkusen(DE)**
Erfinder: **Zeeck, Axel, Prof. Dr.**
**Brüder-Grimm-Allee 22**
**D-3400 Göttingen(DE)**

(54) **Neue Furane und Lactone aus Streptomyceten, Verfahren zu ihrer Herstellung und ihre Verwendung.**

(57) Die Verbindung der allgemeinen Formel

EP 0 337 152 A1

Xerox Copy Centre

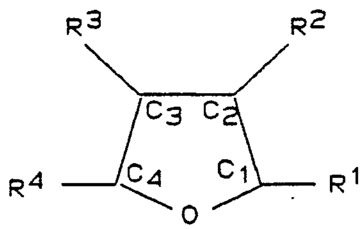

I

in der unabhängig voneinander

$R^1$ Wasserstoff oder eine Oxogruppe,

$R^2$ Methyl oder 1-Hydroxy-ethyl,

$R^3$ Wasserstoff, Methyl oder Rhamnosyloxy-carbonyl

$R^4$ Wasserstoff, 2-Hydroxy-propyl, Acetoxy oder Methyl bedeutet,

wobei die Bindungen zwischen $C_1$ und $C_2$ sowie $C_3$ und $C_4$ Doppelbindungen sein können.

können aus vershiedenen Streptomyceten-Arten hergestellt werden, und besitzen pharmakologische, insbesondere antibiotische Wirkung.

## Neue Furane und Lactone aus Streptomyceten, Verfahren zu ihrer Herstellung und ihre Verwendung

Von H. Zähner ist die Herstellung des antimikrobiell aktiven Keto-acetoxylactons Acetomycin mit Hilfe von Streptomyces ramulosus sp. nov. beschrieben worden [Helvetica Chimica Acta 26, 216 (1958)].

Überraschend wurde nun gefunden, daß Streptomyces spec. DSM 4349, DSM 4355, DSM 4200 und DSM 4211 neue Furane und Lactone mit pharmakologischer, insbesondere antibakterieller Wirkung, bzw. Rhamnose enthaltende Zwischenprodukte, aus denen der Zucker leicht gewonnen werden kann, synthetisieren.

Die Erfindung betrifft somit:

1. Eine Verbindung der allgemeinen Formel I,

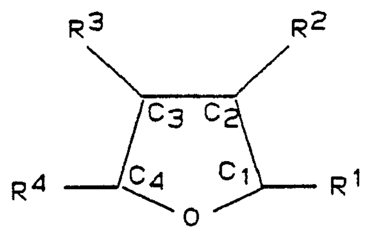

I

in der unabhängig voneinander
$R^1$ Wasserstoff oder eine Oxogruppe,
$R^2$ Methyl oder 1-Hydroxy-ethyl,
$R^3$ Wasserstoff, Methyl oder Rhamnosyloxy-carbonyl
$R^4$ Wasserstoff, 2-Hydroxy-propyl, Acetoxy oder Methyl sein kann,
wobei die Bindungen zwischen $C_1$ und $C_2$ sowie $C_3$ und $C_4$ Doppelbindungen sein können.

2. Ein Verfahren zur Herstellung der Verbindung der allgemeinen Formel I, dadurch gekennzeichnet, daß Streptomyces spec. DSM 4349, DSM 4355, DSM 4200 und DSM 4211, sowie deren Varianten und Mutanten, jeweils einzeln oder in Mischkultur miteinander, in einem Nährmedium kultiviert werden, bis sich die Verbindung der Formel I in der Kultur anhäuft.

3. Die Verwendung der unter 1. charakterisierten Verbindung zur Herstellung von Rhamnose bzw. zur Herstellung von Heilmitteln, insbesondere mit antibiotischer Aktivität.

Im folgenden wird die Verbindung detailliert beschrieben, insbesondere in ihren bevorzugten Ausführungsformen. Ferner wird die Erfindung in den Patentansprüchen definiert.

Die erfindungsgemäß eingesetzten Streptomyceten wurden aus Bodenproben isoliert und bei der Deutschen Sammlung von Mikroorganismen unter den angegebenen DSM-Nummern hinterlegt. Die Hinterlegung erfolgte nach den Regeln des Budapester Vertrags am 5.8.1987, 13.8.1987 und 15.1.1988. Die Mikroorganismen sind wie folgt zu beschreiben:

| | | DSM 4349 | DSM 4355 | DSM 4200 | DSM 4211 |
|---|---|---|---|---|---|
| Sporenfarbe: | | blau/grau | rosa | rot/braun | gelb |
| Sporenkette: | | auseinandergezogene Spirale | gerade | enge Spirale | enge Spirale |
| Sporenoberfläche: | | Stachelig | - | glatt | - |
| Melanin: | | + | + | - | - |
| Pigment: | Substrat-Mycel | Endo: -<br>Exo: - | Endo: -<br>Exo: - | Endo: -<br>Exo: rot | Endo: -<br>Exo: rot |
| | Luft-Mycel | Endo: -<br>Exo: - | Endo: -<br>Exo: - | Endo: braun<br>Exo: - | Endo: -<br>Exo: - |
| | | DSM 4349 | DSM 4355 | DSM 4200 | DSM 4211 |
| Zucker-, Zuckeralkohol-Verwertung | | Arabinose<br>Xylose<br>Rhamnose<br>Raffinose<br>Mannit | nicht getestet | Arabinose<br>Xylose | nicht getestet |
| Säureverwertung: | | Oxalat<br>Malonat | nicht getestet | - | nicht getestet |

In einer Nährlösung, die eine Kohlenstoffquelle und eine Stickstoffquelle, sowie die üblichen anorganischen Salze enthält, produzieren Streptomyces spec. DSM 4349, DSM 4355, DSM 4200 und DSM 4211, einzeln oder in Mischkultur miteinander die obenaufgeführte Verbindung der allgemeinen Formel I, in der $R^1$ bis $R^4$ die obengenannte Bedeutung haben und die Bindungen zwischen $C_1$ und $C_2$ sowie $C_3$ und $C_4$ Doppelbindungen sein können.

DSM 4349 synthetisiert bevorzugt die Verbindung der allgemeinen Formel I, in der $R^1$ und $R^3$ Wasserstoff, $R^2$ Methyl und $R^4$ 2-Hydroxy-propyl bedeutet, wobei die Bindungen zwischen $C_1$ und $C_2$ sowie $C_3$ und $C_4$ Doppelbindungen sind, während DSM 4200 und DSM 4211 bevorzugt die Verbindungen, in denen $R^1$ eine Oxogruppe, $R^2$ 1-Hydroxy-ethyl, $R^3$ Methyl und $R^4$ Wasserstoff oder Acetoxy bedeutet, produzieren sowie die Verbindung, in der $R^1$ Wasserstoff, $R^2$ und $R^4$ Methyl und $R^3$ Rhamnosyloxy-carbonyl ist, wobei die Bindungen zwischen $C_1$ und $C_2$ sowie $C_3$ und $C_4$ Doppelbindungen sind. Mit Hilfe von DSM 4355 kann jede der genannten Verbindungen hergestellt werden.

Anstelle der genannten DSM-Stämme können auch deren Mutanten und Varianten eingesetzt werden, soweit sie mindestens eine dieser Verbindungen synthetisieren. Solche Mutanten können in an sich bekannter Weise durch physikalische Mittel, beispielsweise Bestrahlung, wie mit Ultraviolett- oder Röntgenstrahlen oder chemische Mutagene, wie beispielsweise Methansulfonsäureethylester (Ethylmethansulfonat, EMS) N-Methyl-N′-nitro-N-nitrosoguanidin (MNNG) oder 2-Hydroxy-4-methoxy-benzophenon (MOB) erzeugt werden.

Als bevorzugte Kohlenstoffquellen für die aerobe Fermentation eignen sich assimilierbare Kohlenhydrate und Zuckeralkohole, wie beispielsweise Glucose, Lactose oder D-Mannit und Glycerin sowie kohlenhydrathaltige Naturprodukte, wie Malzextrakt. Als bevorzugte stickstoffhaltige Nährstoffe kommen in Betracht: Aminosäuren, Peptide und Proteine sowie deren Abbauprodukte, wie Peptone oder Tryptone, ferner Fleischextrakte, gemahlene Samen, beispielsweise von Mais, Weizen, Bohnen, Soja oder der Baumwollpflanze, Destillationsrückstände der Alkoholherstellung, Fleischmehle oder Hefeextrakte, aber auch Ammoniumsalze und Nitrate. Außerdem kann die Nährlösung beispielsweise Chloride, Carbonate, Sulfate oder Phosphate der Alkali-oder Erdalkalimetalle, Eisen, Zink und Mangan als zusätzliche anorganische Salze enthalten.

Die Bildung der Furane und Lactone der allgemeinen Formel I verläuft besonders gut in einer Nährlösung, die Glycerin in Konzentrationen von 0,5 bis 6 %, bevorzugt 2 bis 4 %, sowie Caseinpepton in Konzentrationen von 0,1 bis 4 %, bevorzugt 0,5 bis 2 %, enthält jeweils bezogen auf das Gewicht der gesamten Nährlösung. Weitere bevorzugte Nährmedien, insbesondere für DSM 4355 zur Herstellung der Verbindungen, in denen $R^1$ eine Oxogruppe, $R^2$ 1-Hydroxy-ethyl, $R^3$ Methyl und $R^4$ Wasserstoff oder Acetoxy bedeutet, sind solche, die Haferflocken in Konzentrationen von 0,5 bis 6 %, bevorzugt 1 bis 3 %, und Sojamehl in Konzentrationen von 0,1 bis 3 %, bevorzugt 0,3 bis 1 %, bzw. Nährmedien, die Sojamehl und Mannit in Konzentrationen von 0,5 bis 6 %, bevorzugt 1 bis 4 %, enthalten, ebenfalls jeweils bezogen auf das Gewicht des gesamten Nährmediums.

Die Fermentation erfolgt aerob, also beispielsweise submers unter Schütteln oder Rühren in Schüttelkolben oder Fermentern, gegebenenfalls unter Einführen von Luft oder Sauerstoff. Die Fermentation kann in einem Temperaturbereich von etwa 18 bis 40° C, vorzugsweise bei etwa 25 bis 30° C, insbesondere bei 28 bis 30° C erfolgen. Man kultiviert den Mikroorganismus unter den genannten Bedingungen bis die stationäre Phase erreicht ist, etwa 60 bis 100 Stunden, bevorzugt 70 bis 75 Stunden. Die Fermentation läuft im wesentlichen in einem pH-Bereich von ca. 6 bis 8 ab, bzw. in dem bevorzugten Bereich von 6,5 bis 7,5.

Vorteilhaft kultiviert man die Mikroorganismen in mehreren Stufen, d.h. man stellt zunächst eine oder mehrere Vorkulturen in einem flüssigen Nährmedium her, die dann in das eigentliche Produktionsmedium, die Hauptkultur, beispielsweise im Volumenverhältnis 1 : 10, überimpft werden. Die Vorkultur erhält man beispielsweise, indem man ein versportes Mycel in eine Nährlösung überimpft und etwa 48 bis 72 Stunden wachsen läßt. Das versporte Mycel kann erhalten werden, indem man den Stamm etwa 7 Tage auf einem festen oder flüssigen Nährboden, beispielsweise Hefe-Malz-Agar, wachsen läßt.

Der Fermentationsverlauf kann anhand des pH-Wertes der Kultur oder des Mycelvolumens, durch Dünnschichtchromatographie oder Ausprüfen der biologischen Aktivität überwacht werden.

Während der Fermentation der genannten Stämme wird die linksdrehende Verbindung der Formel II

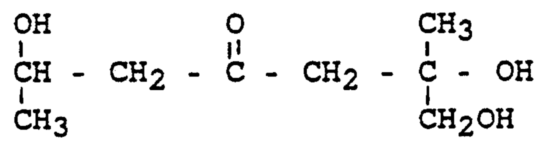

(II)

in optisch reiner Form als Zwischenprodukt gebildet. Die Erfindung betrifft daher auch diese Verbindung und deren Verwendung als Zwischenstufe zur Herstellung der Verbindung der allgemeinen Formel I, in der $R^1$ und $R^3$ Wasserstoff, $R^2$ Methyl und $R^4$ 2-Hydroxypropyl ist.

Die Isolierung der erfindungsgemäßen Furane und Lactone der allgemeinen Formel I aus dem Kulturmedium erfolgt nach bekannten Methoden unter Berücksichtigung der chemischen, physikalischen und biologischen Eigenschaften der Produkte. Zum Testen der Antibiotika-Konzentration im Kulturmedium oder in den einzelnen Isolierungsstufen kann die Dünnschichtchromatographie, beispielsweise auf Kieselgel mit Chloroform/Methanol als Laufmittel verwendet werden, wobei die Menge der gebildeten Antibiotika zweckmäßig mit einer Eichlösung verglichen wird.

Die Lactone bzw. Furane der allgemeinen Formel I liegen im Mycel bzw. in der Kulturbrühe vor. Sie können aus der unfiltrierten Kulturbrühe mit einem mit Wasser nicht oder nur wenig mischbaren organischen Lösemittel, wie Chloroform oder Ethylacetat, extrahiert werden. Da sie sich jedoch nur zu einem geringen Teil im Mycel befinden, ist es vorteilhaft, die Kulturbrühe vom Mycel zu trennen, beispielsweise durch Zentrifugieren oder Filtrieren, vorzugsweise unter Zugabe von Filterhilfsmitteln.

Die Verbindungen der Formel I können dann aus dem Überstand bzw. Filtrat isoliert werden, zweckmäßig im leicht sauren bis neutralen pH-Bereich, vorzugsweise bei pH 6 bis 7. Dazu verwendet man zweckmäßig mit Wasser wenig oder nicht mischbare organische Lösemittel, insbesondere chlorierte Kohlenwasserstoffe, wie Chloroform oder Methylenchlorid oder Ester wie Ethylacetat oder Aceton. Die Extrakte werden, gegebenenfalls nach Eindampfen und Aufnehmen in einem polaren organischen Lösemittel, durch Fällen mit einem unpolaren Lösemittel, zweckmäßig einem Kohlenwasserstoff wie Petrolether, von stark lipophilen Bestandteilen befreit, die bis zu etwa 80 % des Gesamtextraktes ausmachen können. Aus dem Rückstand können die Lactone und Furane durch Chromatographie isoliert werden.

Zur weiteren Isolierung aus dem entfetteten Rohextrakt wird dieser zweckmäßig über eine Kieselgelsäule gereinigt, wobei sich als Fließmittel Gemische aus niedermolekularen Chlorkohlenwasserstoffen und Alkanolen, beispielsweise Chloroform und Methanol im Volumenverhältnis 4 : 1 oder Ethylacetat und Hexan im Volumenverhältnis 1 : 2 bewährt haben. Die adsorbierten Komponenten werden nacheinander eluiert.

Zur Isolierung der reinen Stoffe können die üblichen Verfahrensschritte Verwendung finden, wie Chromatographie, Gelfiltration oder Fällung aus ihren Lösungen in geeigneten organischen Lösemitteln. Besonders bewährt hat sich eine Chromatographie an Kieselgel, wobei als Laufmittel eine Mischung aus Ethylacetat und Hexan in einem Volumenverhältnis von beispielsweise 1 : 2 Verwendung findet.

Die linksdrehende Verbindung der Formel II in optisch reiner Form kann ebenfalls auf dem im vorangegangenen beschriebenen Weg isoliert werden.

Die Lactone und Furane sind ölig und gut in Methanol, Aceton, DMSO, Dioxan und Chloroform löslich, jedoch nicht in Wasser und Alkanen. Die Substanzen sind im festen Zustand wie auch als Lösung im pH-Bereich von 3 bis 9, insbesondere von 5 bis 8, stabil und lassen sich somit in galenische Zubereitungen einarbeiten.

Die antibakterielle Wirkung kommt besonders gegen Staphylococcen und Streptococcen zur Geltung, wie beispielsweise im Platten-Agardiffusionstest in vitro (10 µl/Testrondell, 6 mm Durchmesser) gezeigt werden kann. Verschiedene Keime, die sich gegenüber einer minimalen Hemmkonzentration im Bereich von > 10 bis < 50 µg/ml sensitiv zeigen, sind beispielsweise: Staphylococcus aureus, Streptococcus pyogenes und Streptococcus faecium.

**Beispiele:**

**1. a) Herstellung einer Sporensuspension des Produzentenstammes:**

100 ml Nährlösung (4 g Hefeextrakt, 10 g Malzextrakt, 4 g Glucose, 1 l Leitungswasser, pH-wert vor dem Sterilisieren 7,3) in einem 500 ml Erlenmeyerkolben werden mit dem Stamm DSM 4355 beimpft und 72 Stunden bei 27° C und 120 UpM auf der rotierenden Schüttelmaschine inkubiert. Anschließend werden 20 ml Kulturflüssigkeit in einem 500 ml Erlenmeyerkolben mit dem Nährboden der oben genannten Zusammensetzung, dem 20 g Agar/l zur Verfestigung zugegeben wurde, gleichmäßig verteilt und dekantiert. Die Kulturen werden 10 bis 14 Tage bei 27° C inkubiert. Die nach dieser Zeit entstandenen Sporen eines Kolbens werden mit 500 ml entionisiertem Wasser, das einen Tropfen eines handelsüblichen nichtionischen Tensids enthält (Triton X 100, Fa. Serva) abgeschwemmt, sofort weiterverwendet oder bei -22° C aufbewahrt.

Mit den Stämmen DSM 4349, DSM 4200 und DSM 4211 kann hier und im folgenden analog verfahren werden.

**b) Herstellung einer Kultur bzw. Vorkultur des Produzentenstammes im Erlenmeyerkolben.**

Ein 500 ml Erlenmeyerkolben mit 100 ml einer Nährlösung der Zusammensetzung 2 % Fleischmehl, 10 % Malzextrakt, 1 % Calciumcarbonat und Wasser ad 100 ml (pH 7,2 vor dem Autoklavieren) wird mit einer auf einem Schrägröhrchen gezogenen Kultur oder mit 0,2 ml Sporensuspension angeimpft und auf einer Schüttelmaschine bei 120 UpM und 27° C inkubiert. Die maximale Antibiotikumproduktion ist nach 72 Stunden erreicht. Zum Animpfen von 10 und 100 l Fermentern genügt eine 48 Stunden alte Submerskultur (5 %) aus der gleichen Nährlösung.

**2. Herstellung der Furane und Lactone**

Ein 10 l Fermenter wird unter folgenden Bedingungen betrieben:

| | |
|---|---|
| Nährmedium: | 30 g/l Glycerin<br>2 g/l Caseinpepton<br>1 g/l $K_2HPO_4$<br>1 g/l NaCl<br>0,5 g/l $MgSO_4 \bullet 7H_2O$<br>5 ml/l Spurenelementlösung |
| Spurenelemente: | 3 g/l $CaCl_2 \bullet 2H_2O$<br>1 g/l $FeC_6O_7H_5$<br>0,2 g/l $MnSO_4$<br>0,1 g/l $ZnCl_2$<br>0,025 g/l $CuSO_4 \bullet 5H_2O$<br>0,02 g/l $Na_2B_4O_7 \bullet 10H_2O$<br>0,004 g/l $CoCl_2$<br>0,01 g/l $Na_2MoO_4 \bullet 2H_2O$ |
| Inkubationszeit: | 72 Stunden |
| Inkubationstemperatur: | 30° C |
| Rührergeschwindigkeit: | 250 UpM |
| Belüftung: | 4 l Luft/min. |

Durch wiederholte Zugabe weniger Tropfen ethanolischer Polyollösung kann die Schaumentwicklung unterdrückt werden. Das Produktionsmaximum wird nach ca. 60-80 Stunden (pH = 5,3) erreicht. Die Ausbeuten liegen bei ca. 10 mg/l.

### 3. Isolierung der Furane und Lactone

Nach der Fermentation wird die Kulturbrühe unter Zusatz von 2 % Celite als Filterhilfsmittel filtriert. Das Mycel wird mit Aceton extrahiert, die organische Phase eingedampft und der wäßrige Rückstand mit Ethylacetat extrahiert. Das Kulturfiltrat wird ebenfalls bei pH 7 erschöpfend mit Ethylacetat extrahiert. Dieser Extrakt wird mit dem des Mycels vereinigt, getrocknet und eingedampft. Das Rohprodukt wird an einer Kieselgelsäule (Kieselgel 60, Fa. Macherey-Nagel) mit einer Mischung aus Ethylacetat und Hexan im Verhältnis 1 : 2 (v:v) chromatographiert.

### 4. a) Charakterisierung der Verbindung

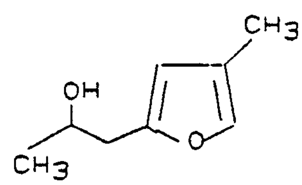

Dünnschichtchromatographie:
Kieselgel 60, $F_{254}$: Chloroform/Methanol (9 : 1, v:v): Rf 0,40
EI-MS (70 eV): m/e = 140 ($M^+$, 20 %); 97 (100 %)
entsprechend $C_8H_{12}O_2$ (140,18)
UV (Methanol): $\lambda_{max}$ ($\epsilon$) = 224 (3000) nm
UV (Methanol/HCl): $\lambda_{max}$ ($\epsilon$) = 218 (2700) nm
UV (Methanol/NaOH): $\lambda_{max}$ ($\epsilon$) = 219 (6400) nm
$^1$H-NMR (200 MHz, $CDCl_3$): $\delta$ = 1,22 (d,3H,J = 6 Hz, 8-$H_3$); 2,00 (s,3H,9-$H_3$); 2,7 (m,2H,6-$H_2$); 3,7 (m,1H,7-H); 4,1 (m,1H,tauscht aus, 7-OH); 6,00 (s,1H,3-H); 7,1 (s,1H,5-H)ppm.

### b) Charakterisierung der Verbindung:

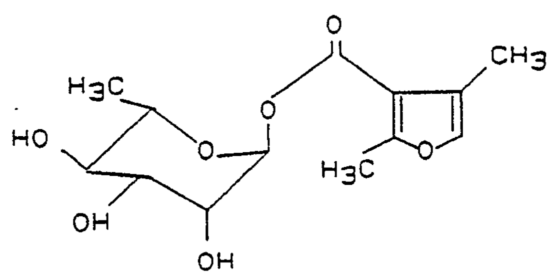

Dünnschichtchromatographie:
Kieselgel 60, $F_{254}$ Chloroform/Methanol (9:1, v:v): Rf 0,28
EI-MS (70 eV): m/e = 286 ($M^+$, 10 %, $C_{13}H_{18}O_7$, Hochauflösung) 140 (74 %, $C_7H_8O_3$, Hochauflösung); 123 (100 %, $C_7H_7O_2$, Hochauflösung).
IR (KBr): 3200-3500, 2978, 2930, 1705, 1608 $cm^{-1}$.
$^1$H-NMR (200 MHz, $CDCl_3$): $\delta$ = 1,28 (d,3H,J = 5,9 Hz,6′-H3); 2,06 (d,3H,J<1 Hz, 7-$H_3$); 2,47 (s, 3H,6-$H_3$); 3,57 (t,1H, J = 9,4 Hz, 4′-H); 3,70 (dq,1H,J = 9,4/5,9 Hz, 5′-H); 3,81 (dd,1H,J = 9,4/3,4 Hz,3′-H); 4,05 (dd,1H,J = 3,4/1,6 Hz, 2′-H); 6,20 (s,breit,1H,1′-H); 6,97 (s,1H,5-H)ppm.
$^{13}$C-NMR (50,3 MHz,$CD_3OD$): $\delta$ = 10,6 q; 14,7 q; 18,1 q (C-6′); 71,3 d; 72,3 d; 72,6 d; 73,3 d; 95,1 d (C-1′); 113,9 s (C-4); 122,1 s (C-3); 139,6 d(C-5); 162,4 s (C-2); 163,9 s (C-8)ppm.
$[\alpha]_D^{20}$ : (c = 0,5, Methanol) -30°

**c) Charakterisierung der Verbindungen**

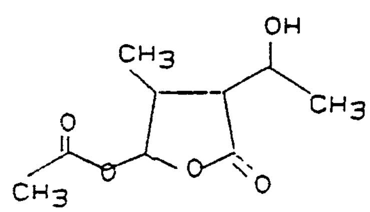 A; 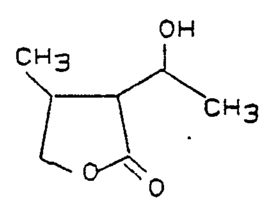 B;

Die Verbindungen der obengenannten Formeln liegen als Mischung vor im Verhältnis 2:1 (A:B)
Dünnschichtchromatographie:
Kieselgel 60, $F_{254}$ Chloroform/Methanol (9:1,v:v): Rf. 0,57
IR (KBr): 3200-3600, 1785, 1760 $cm^{-1}$
$^1$H-NMR (200 MHz, $CD_3OD$):

Signale Verbindung A:

$\delta$ = 1,21 (d,3H,J = 7 Hz); 1,31 (d,3H,J = 6,6 Hz);
2,12 (s,3H,Acetyl-$CH_3$); 2,50 (dd,1H,J = 10,2/3,3 Hz,3-H);
2,9 (m,1H,4-H); 4,28 (m,1H,6-H); 6,52 (d,1H,5-H)ppm.

Signale Verbindung B:

$\delta$ = 1,20 (d,3H,J = 7 Hz); 1,30 (d,3H,J = 6,6 Hz); 2,26 (dd, 1H,J = 8/3,8 Hz,3-H); 2,75 (m,1H,4-H); 3,80 (t,1H,J = 8 Hz, 5-$H_a$); 4,43 (t,1H,J = 8 Hz,5-$H_b$)ppm.
$^{13}$C-NMR (50,3 MHz,$CD_3OD$):

Signale Verbindung A:

$\delta$ = 14,1 g; 20,6 g; 21,9 g; 35,2 d; 52,2 d; 66,7 d; 96,3 d; 171,0 s; 178,4 s ppm.

Signale Verbindung B:

$\delta$ = 19,1 g; 21,8 g; 31,5 d; 55,4 d; 67,2 d; 74,7 t; 180,9 s ppm.

**d) Charakterisierung der Verbindung II**

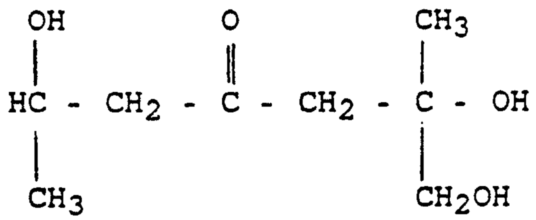

II

$C_8H_{16}O_4$ (176.2)
Dünnschichtchromatographie:
Kieselgel 60, $F_{254}$ $CHCl_3$/Methanol (9:1, v.v): $R_f$ = 0,23
$^1$H-NMR (200 MHz, $CDCl_3/CD_3OD$): $\delta$ = 1,15 (s,3H,8-$H_3$); 1,2 (d,3H,1-$CH_3$); 2,65 (d,1H,J = 12,5Hz,5-$H_b$); 2,7 (dd,3-H); 2,75 (d,1H J = 12,5Hz,5-$H_a$); 3,85 (m,2H,7$H_2$); 4,2 (q,1H, 2-H) ppm.
$^{13}$C-NMR (50,3 MHz, $CDCl_3/CD_3OD$): $\delta$ = 23,3 q ($CH_3$); 23,4 q ($CH_3$); 49,6 t ($CH_2$); 52,9 t ($CH_2$); 63,5 t ($OCH_2$); 68,7 d (CH); 72,1 d (CH); 211,2 s (CO) ppm.
Optischer Drehwert $[\alpha]_D^{20}$ : -12 (c = 1 in $CHCl_3$)

**Ansprüche**

1. Die Verbindung der allgemeinen Formel I,

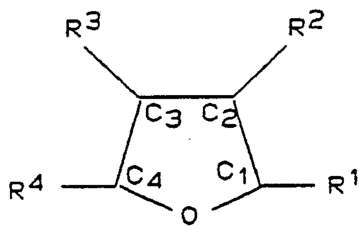

in der unabhängig voneinander
$R^1$ Wasserstoff oder eine Oxogruppe,
$R^2$ Methyl oder 1-Hydroxy-ethyl,
$R^3$ Wasserstoff, Methyl oder Rhamnosyloxy-carbonyl
$R^4$ Wasserstoff, 2 Hydroxy-propyl, Acetoxy oder Methyl bedeutet,
wobei die Bindung zwischen $C_1$ und $C_2$ sowie $C_3$ und $C_4$ Doppelbindungen sein können.

2. Verfahren zur Herstellung der Verbindung der allgemeinen Formel I nach Anspruch 1 und der linksdrehenden Verbindung der Formel II in optisch reiner Form,

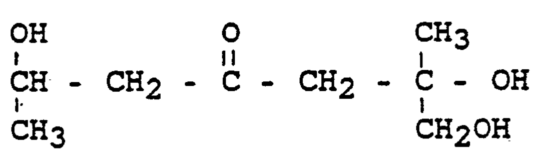

II

dadurch gekennzeichnet, daß Streptomyces spec. DSM 4349, DSM 4355, DSM 4200 und DSM 4211 sowie deren Varianten und Mutanten, einzeln oder in Mischkultur miteinander in einem Nährmedium kultiviert werden, bis sich jeweils die Verbindung der allgemeinen Formel I und die linksdrehende Verbindung der Formel II in optisch reiner Form in der Kultur anhäuft.

3. Verfahren nach Anspruch 2, dadurch gekennzeichnet, daß die Nährlösung Glycerin in Konzentrationen von 0,5 bis 6 % und Caseinpepton in Konzentrationen von 0,1 bis 4 % enthält, bzw. Haferflocken in Konzentrationen von 0,5 bis 6 % und Sojamehl in Konzentrationen von 0,1 bis 3 % bzw. Sojamehl und Mannit in Konzentrationen von 0,5 bis 6 %, jeweils bezogen auf das Gesamtgewicht des Nährmediums, enthält.

4. Verfahren nach Anspruch 2 oder 3, dadurch gekennzeichnet, daß die Mikroorganismen in einem Temperaturbereich von 18 bis 40 °C kultiviert werden.

5. Verfahren nach Anspruch 4, dadurch gekennzeichnet, daß in einem Temperaturbereich von 25 bis 30 °C kultiviert wird.

6. Verfahren nach einem oder mehreren der Ansprüche 2 bis 5, dadurch gekennzeichnet, daß die Mikroorganismen kultiviert werden, bis die stationäre Phase erreicht ist.

7. Verwendung der Verbindung der allgemeinen Formel I nach Anspruch 1 zur Herstellung von Rhamnose.

8. Verwendung der Verbindung der allgemeinen Formel I nach Anspruch 1 zur Herstellung von Heilmitteln.

9. Verwendung der Verbindung der allgemeinen Formel I nach Anspruch 1 zur Herstellung eines antibiotischen Heilmittels.

10. Streptomyces spec. DSM 4349, DSM 4355, DSM 4200 und DSM 4211 sowie deren Varianten und Mutanten, sofern sie die Verbindung der allgemeinen Formel I nach Anspruch 1 synthetisieren.

11. Verwendung der nach Anspruch 2 hergestellten Verbindung II als Zwischenprodukt zur Herstellung der Verbindung der allgemeinen Formel I nach Anspruch 1, in der $R^1$ und $R^3$ Wasserstoff, $R^2$ Methyl und $R^4$ 2-Hydroxy-propyl ist.

Europäisches Patentamt

# EUROPÄISCHER RECHERCHENBERICHT

Nummer der Anmeldung

EP 89 10 4761

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | BULLETIN DE LA SOCIETE CHIMIQUE DE FRANCE, Nr. 9, September 1972, Seiten 3616-3524, Paris, FR; J. BOGNER et al.: "Etude conformationnelle de tétrahydrofurannes méthylés" * Seite 3622, Verbindungen 1,2a,2b; Seite 3623, Verbindungen 4a,4b * --- | 1 | C 07 D 307/32<br>C 07 D 307/42<br>C 07 H 13/10<br>C 07 H 7/02<br>A 61 K 31/34<br>A 61 K 31/71<br>C 12 N 1/20<br>C 12 P 17/04<br>C 12 P 19/02 //<br>(C 12 P 17/04<br>C 12 R 1:465)<br>(C 12 P 19/02<br>C 12 R 1:465)<br>(C 12 N 1/20<br>C 12 R 1:465) |
| X | BULLETIN OF THE CHEMICAL SOCIETY OF JAPAN, Band 60, Nr. 1, Januar 1987, Seiten 229-240, The Chemical Society of Japan, Tokyo, JP; K. YOSHIDA et al.: "Concurrent anodic cyanation and methoxylation of methylated furans. Oxidation potential and reactivity, and stereochemical control of addition" * Seite 238, Tabelle 6 * --- | 1 | |
| X | JOURNAL OF ORGANIC CHEMISTRY, Band 42, Nr. 6, 18. März 1977, Seiten 1089-1090, Gaston, US; A. ZAMOJSKI et al.: "Synthesis of 3-substituted furans" * Seite 1089, Verbindung 8 * --- | 1 | **RECHERCHIERTE SACHGEBIETE (Int. Cl.4)** |
| X | TETRAHEDRON LETTERS, Band 26, Nr. 39, 1985, Seiten 4815-4818, Pergamon Press Ltd, Oxford, GB; S.G. DAVIES et al.: "Chiral discrimination in the reactions of the enolate E-[(n5-c5h5)Fe(CO)(PPh3)COCHMe]-Li+ with cis and trans but-2-ene oxides in the presence of BF3.OEL3" * Seite 4816, Verbindungen 6,7 * --- -/- | 1 | C 07 D 307/00<br>C 07 H 13/00<br>C 07 H 7/00<br>C 12 P 17/00 |

Der vorliegende Recherchenbericht wurde für alle Patentansprüche erstellt

| Recherchenort | Abschlußdatum der Recherche | Prüfer |
|---|---|---|
| DEN HAAG | 23-06-1989 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument
& : Mitglied der gleichen Patentfamilie, übereinstimmendes Dokument

EPO FORM 1503 03.82 (P0403)

Europäisches Patentamt

# EUROPÄISCHER RECHERCHENBERICHT

Nummer der Anmeldung

EP 89 10 4761

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | JOURNAL OF THE CHEMICAL SOCIETY, CHEMICAL COMMUNICATIONS, 15. April 1985, Seiten 511-512, Londen, GB; H. ALPER et al.: "Palladium-catalysed convension of alkenols into five- and six-membered ring lactones at room temperature and atmospheric pressure" * Seite 512, Tabelle 1 * --- | 1 | |
| X | BULLETIN OF THE CHEMICAL SOCIETY OF JAPAN, Band 52, Nr. 8, August 1979, Seiten 2389-2393, Tokyo, JP; I. MATSUDA et al.: "A simple synthesis of alpha-Ylidene beta-Lactones from alpha-Trimethylsiloxy nitriles" * Seiten 2389-2390, Verbindungen 7d,7e * --- | 1 | |
| X | BULLETIN OF THE CHEMICAL SOCIETY OF JAPAN, Band 57, Nr. 3, März 1984, Seiten 897-898, Tokyo, JP; T. IKARIYA et al.: "Regioselective hydrogenation of unsymmetrically substituted cyclic anhydrides catalyzed by ruthenium complexes with phosphine ligands" * Seite 897, Schema 2 * --- | 1 | RECHERCHIERTE SACHGEBIETE (Int. Cl.4) |
| A | CHEMICAL ABSTRACTS, Band 82, Nr. 7, 17. Februar 1975, Seite 412, Nr. 43107a, Columbus, Ohio, US; T. MUKAIYAMA et al.: "Convenient synthesis of the antibiotic botryodiplodin", & CHEM. LETT. 1974, (10), 1181-4 ----- | 1,8,9 | |

Der vorliegende Recherchenbericht wurde für alle Patentansprüche erstellt

| Recherchenort | Abschlußdatum der Recherche | Prüfer |
|---|---|---|
| DEN HAAG | 23-06-1989 | ALLARD M.S. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)